# EUROPEAN PATENT SPECIFICATION

(11) **EP 2 510 381 B1**
(45) Date of publication and mention of the grant of the patent: **08.01.2014**
(21) Application number: 10788030.4
(22) Date of filing: 16.11.2010
(51) Int. Cl.: G01S 7/52, G01S 7/531

(54) **METHOD OF RE-SAMPLING ULTRASOUND DATA**
VERFAHREN ZUR NEUABTASTUNG VON ULTRASCHALLDATEN
PROCEDE DE REECHANTILLONNAGE DE DONNEES ULTRASONORES

(30) Priority: 16.11.2009 WO PCT/EP2009/065246
(43) Date of publication of application: 17.10.2012
(73) Proprietor: Advanced Medical Diagnostics Holding S.A., 1410 Waterloo (BE)
(72) Inventor: BORE, Chris, Woking Surrey GU22 0JZ (GB); NIR, Dror, B-1410 Waterloo (BE); NIR, Rina, B-1410 Waterloo (BE); SULIGA, Marek, B-1160 Auderghem (BE)
(74) Representative: Brants, Johan P.E.
(86) International application number: PCT/EP2010/067529
(87) International publication number: WO 2011/058186

(56) References cited:
- US-A- 4 471 449
- US-A- 5 538 004
- US-A- 5 546 807
- RASCHE V ET AL: "Resampling of Data Between Arbitrary Grids Using Convolution Interpolation", IEEE TRANSACTIONS ON MEDICAL IMAGING, IEEE SERVICE CENTER, PISCATAWAY, NJ, US, vol. 18, no. 5, 1 May 1999 (1999-05-01), XP011035860, ISSN: 0278-0062
- DUAN Q ET AL: "Assessment of visual quality and spatial accuracy of fast anisotropic diffusion and scan conversion algorithms for real-time three-dimensional spherical ultrasound", PROC. SPIE,, vol. 5373, 7 May 2004 (2004-05-07), pages 331-342, XP007912505, DOI: 10.1117/12.535813

## Description

### Field of the invention

The present invention relates to multi-dimensional filtering of ultrasound scan data for antialiasing or reconstruction for the purpose of re-sampling with the aim of scaling, interpolation and decimation.

### Background of the invention

Ultrasound diagnostic imaging systems are in widespread use by cardiologists, obstetricians, radiologists and others for examinations of the heart, a developing fetus, internal abdominal organs and other anatomical structures.

Conventional ultrasound imaging systems comprise an array of ultrasonic transducers which transmit waves of ultrasound energy into the body, receiving ultrasound echo signals that have been scattered from tissue upon which the waves impinge. The transmitted waves are shaped and timed from each transducer so that they form ultrasound beams (also called 'lines of sight') that radiate from the transducer array surface (this process is called 'beam forming'): the received echo signals are also processed to reinforce the effect of beam forming, so that the received signals represent the ultrasound scattering measured along the lines of sight.

The received signals are sampled and digitized to provide an array of samples that represent the ultrasound scattering at spatial locations within the scanned volume. The samples provide measurements of the signal at specific positions (usually positions in space and time) separated by intervals (usually intervals of time, spatial distance or angle).

Co-ordinate systems that are common or convenient for analysis, processing or rendering of ultrasound data are often different from the co-ordinate systems that are defined by the original scan geometry. So the original sample data must be re-sampled and processed to provide an' array of samples at uniformly spaced positions along the new target coordinate axes.

For example the most common co-ordinate system for processing and rendering of the ultrasound data is rectangular Cartesian (because displays tend to be rectangular and because most processing algorithms assume a rectangular Cartesian input) so the data must be re-sampled to be evenly spaced within a rectangular Cartesian co-ordinate system in preparation for processing or rendering. A rectangular Cartesian sampled data co-ordinate system is also convenient for processing and analysis because each sample represents the same volume. So in this case the original sample data must be re-sampled to provide an array of samples at uniformly spaced positions along rectangular Cartesian co-ordinate axes.

A method according to the preamble of claim 1 is known from US 4,471,449.

It is common to desire that a set of samples of an ultrasound image be used to calculate a larger or smaller set whose sample spacings may be smaller or larger than those of the original set, and whose sample positions may lie between the input samples, and where some original samples may no longer be desired, and this requires re-sampling.

The process of re-sampling should satisfy the requirements of Nyquist-Shannon Sampling Theory (hereinafter called Sampling Theory), which defines the conditions under which a set of samples can completely determine the underlying continuous signal. In particular Sampling Theory requires that during re-sampling, input samples must be filtered using filters whose characteristics depend on the original and the new sample spacings.

Current methods of designing filters for re-sampling in ultrasound use filters that cannot be shown to satisfy the requirements of Sampling Theory. Current methods of filtering and re-sampling in ultrasound design filters relative to the target co-ordinate system, where the original samples are not uniformly spaced so Sampling Theory cannot be directly applied.

Current methods of designing filters for re-sampling in ultrasound have the aim of producing a pleasing visual display. There is no formal definition of 'pleasing' but this usually means a visual display tends to show smooth areas and well-defined boundaries. The genuine underlying data are distorted in order to render this visually pleasing display, so valuable information is lost and false artifacts may be introduced. Such filters are designed through arguments on ill-defined effects such as 'smoothing' and are not characterized by direct applications of Sampling Theory. In particular the mathematical requirements of Sampling Theory are not satisfied, because a pleasing visual display does not require that the underlying signal be completely determined.

Some current methods of filtering for re-sampling in ultrasound use non-linear filters (such as 'median' filters') whose characteristics cannot be calculated directly as applied to Sampling Theory and so which cannot be shown to satisfy the requirements of Sampling Theory.

There is therefore a need to develop new methods for designing and implementing linear filters for re-sampling ultrasound data, respecting the requirements of Sampling Theory so that the data are not distorted and so that valuable information is not lost. It is accordingly, an object of the present invention to provide a novel method and system for designing and implementing linear filters for re-sampling of ultrasound scan data. It is also an object of the present invention to overcome or ameliorate at least one of the disadvantages of the art, or to provide a useful alternative thereto.

### Summary of the invention

The present invention relates to filtering and re-sampling of ultrasound scan data that spans an n-dimensional volume (hereafter called "nD"), for example (but not limited to) a 3D spatial volume. In particular, the present invention relates to re-sampling the ultrasound scan data from co-ordinate geometries in which the samples are uniformly spaced along each axis in the units appropriate to that axis, into a different co-ordinate space where the original samples are not uniformly spaced, but where the new target samples will be uniformly spaced. For example, the invention relates to re-sampling from samples that are uniformly distributed along the axes of a non-Cartesian co-ordinate system that naturally matches the scan geometry, to samples that are uniformly distributed along the axes of a rectangular Cartesian co-ordinate system. In an embodiment, this re-sampling uses anti-alias and reconstruction filters that are designed in the original scan co-ordinate system.

The present invention relates to a method of re-sampling ultrasound scan data in such a way that the underlying signal remains completely determined according to the requirements of Nyquist-Shannon Sampling Theory, and whose effects may be calculated readily using linear mathematical models.

In particular, the present invention relates to a method of re-sampling ultrasound scan data according to claim 1.

The present method has the advantage that the underlying signal remains completely determined and may be further processed and analyzed without distortion or loss of information. This is because the method allows the filters to be designed by direct application of Sampling Theory.

In contrast with the present invention, prior art methods design re-sampling filters in the desired target co-ordinate system, in which the original samples are spaced non-uniformly and so filter design using Sampling Theory (which assumes uniform sample spacing) is not possible: so these filters cannot be shown not to result in distortion or loss of information. The present inventors have shown that by directly applying Sampling Theory and Shannon's method for reconstruction the underlying signal remains completely determined and may be further processed and analyzed without distortion or loss of information.

The present method has the further advantage that it can retain maximum information for minimum data size, and so allows storage requirements and computation to be kept to a minimum. This is because the minimum number of samples necessary to completely determine the underlying signal, and the necessary filter designs, can be calculated directly by application of Sampling Theory and filter length estimation (for example Kaiser's method).

In contrast with the present invention, prior art cannot directly apply Sampling Theory to calculate the minimum number of samples necessary to completely determine the underlying signal, so that either more samples may be used than are necessary, or the signal may be distorted or information may be lost.

In addition, the present method has the advantage of being mathematically correct and satisfying Sampling Theory, and so can be subject to rigorous mathematical analysis. Also, the present method has the advantage of offering a computationally efficient implementation by direct application of an nD filter kernel using a polyphase implementation, because of the uniform spacing of the target samples.

By contrast, prior art methods have a non-uniform spacing of original samples relative to the filter kernel which complicates the filter implementation.

The present invention also provides an apparatus and system for re-sampling ultrasound scan data from various original n-dimensional scan geometries and sample spaces, to a uniform n-dimensional sample grid (often, but not only, rectangular Cartesian) that is convenient for further processing and that reduces the amount of data while retaining the original information according to the measures of Sampling Theory and the physical characteristics of the ultrasound scanning process. The present invention greatly simplifies the display, extraction of features from, and analysis of n-dimensional ultrasound scan data by providing an n-dimensional sample space that is convenient to process and that reduces the amount of data to be processed without undue loss of genuine information.

The present invention in particular provides an apparatus for re-sampling ultrasound scan data according to claim 14.

The present invention also provides an ultrasound processing system according to claim 15.

### Detailed description of the invention

The present invention relates to a method of re-sampling ultrasound scan data in such a way that the underlying signal remains completely determined according to the requirements of Nyquist-Shannon Sampling Theory, and whose effects may be calculated readily using linear mathematical models. For example standard methods of sample rate conversion may be used. Suitable standard methods of sample rate conversion are described in Crochiere and Rabiner, "Multirate Digital Signal Processing", ISBN 0-13-605162-6).

Shannon's Sampling Theorem (Claude E Shannon, "Communication in the presence of noise", Proc IRE, vol 37, No 1, Jan 1949, reprinted in Proc IEEE, Vol 86, No. 2, February 1998) states that a band-limited signal is completely determined by samples spaced at uniform intervals (Oppenheim and Schafer, 'Discrete Time Signal Processing, Prentice Hall, ISBN 0-13-216771-9).That is, if the signal is represented by a function f(t) whose Fourier Transform contains no frequencies higher than W, then the signal is completely determined by its ordinates (samples) at a series of points spaced W/2 apart. The spacing W/2 is called the Nyquist interval and is the largest spacing between samples for which the signal is completely determined. Thus the Nyquist interval spacing represents the sample spacing for which the minimum number of samples, and hence the minimum amount of data, completely determine the signal. Moreover, Sampling Theory is proved for the case where the sample spacing is uniform (that is, all samples are spaced an equal distance apart). As used herein the term "band-limited" means the signal's Fourier transform or power spectral density is zero above a certain finite frequency (that is, the signal's Fourier Transform or power spectral density has finite support). As used herein the term "Completely determined" means that the value of the original signal at any coordinate may be reconstructed from the samples.

According to the invention during re-sampling, input samples are filtered using reconstruction filters whose characteristics depend on the original and the new sample spacings, and whose design must follow Shannon's specification. Suitable reconstruction filters are described for example in Claude E Shannon, Proc IRE, vol 37, No 1, Jan 1949, and in Oppenheim and Schafer, 'Discrete Time Signal Processing, Prentice Hall, ISBN 0-13-216771-9.

As used herein the term "re-sampling" refers to the process of calculating (from the original data samples) the samples that would have been obtained at the new desired sample positions.

In an embodiment, the present method comprises (a) the step of obtaining sampled ultrasound scan data acquired from a beam forming system.

The sample positions and units are determined by the probe and scan geometry, and are most easily described using a co-ordinate system that matches the scan geometry and within which the samples are uniformly spaced along each of the co-ordinate axes, according to the units that are natural to that axis. For example a probe and scan geometry that generates beams radiating in three dimensions from a common center is most easily described using a spherical polar co-ordinate system: the radial dimension being aligned with the axis of the ultrasound lines of sight (also referred herein as the 'axis' dimension) and with the two angular dimensions representing the angular positions of the beams within a frame (also referred herein as the 'azimuth' dimension) and the angular position of frames (also referred herein as the 'elevation' dimension).

The next steps in the present method comprise: (b) defining desired target sample positions in a target n-dimensional co-ordinate system, that are uniformly spaced along each axis when measured in units appropriate to that axis; and (c) mapping the target sample positions into the original co-ordinate system.

As used herein the term "mapping" means expressing the target sample positions using the coordinate system of the original sample co-ordinate system. The original signal at each of these mapped target sample positions may now be reconstructed using a reconstruction filter designed according to Shannon's or another standard method. The reconstruction filter is designed in the original scan co-ordinate system.

The next step in the present method comprises: (d) quantizing the positions of the mapped target samples so that they fall on simple exact integer sub-spacings between the original sample positions and so may be described as having uniform spacings along each axis of the original scan co-ordinate system when measured in units that are appropriate to that axis. Each target sample now has a position relative to its neighbors ('relative position' means the difference between the target co-ordinates and the coordinates of each of its nearest neighbors). Each position of the target sample relative to its neighbors may require a different reconstruction interpolating filter. This means that potentially a large number of reconstruction filters would be needed - one for each unique relative target sample position. However if the calculated target sample positions are quantized (meaning, approximated to a finite numeric precision) in the original co-ordinate system then the number of unique relative positions, and thus the number of unique reconstruction filters required, may be limited. For example if the original scan coordinates are spaced at integer multiples of a separation d, then if the target sample coordinates are quantized to a numeric precision of one decimal place then there will be only 10 relative positions and thus only 10 required interpolation filters.

As used herein the term "quantizing" means converting the position, expressed as a set of co-ordinates in some numeric precision, to a lower numeric precision. "Quantizing the positions" here means, calculating the co-ordinates of the target samples (in the original scan geometry) to some numeric precision, and then converting to a lower numeric precision. For example if the co-ordinates are calculated to 32-bit IEEE floating point numeric precision then they may be quantized to 16-bit integer, or to 16-bit fractional fixed point precision.

The quantizing of position of step (d) reduces the number of different reconstruction filter kernels of step (e) that are required. Each position of the target sample relative to its neighbors requires a different reconstruction interpolating filter. Quantizing the positions reduces the number of relative positions and thus the number of interpolation filters. For example if the original scan co-ordinates are spaced at integer multiples of a separation d, then if the target sample co-ordinates are quantized to a numeric precision of one decimal place then there will be only 10 unique interpolation filters.

In an embodiment, not all of the quantized points may actually be used later, i.e. only part of these potential quantized points are needed (or used). For example, the full grid of quantized points may be a superset of the target grid.

The next step in the present method comprises: (e) designing n-dimensional linear filter kernels, preferably designing a set of n-dimensional liner filter kernels (preferably one for each different target sample position relative to the original sample positions of its nearest neighbors), according to application of Sampling Theory to perform anti-aliasing or reconstruction, based on the original sample spacings and the newly desired target sample positions in their respective n-dimensional spaces (these spacings having been rendered uniform in both cases through the position quantization step), the design could optionally be such that the filters are separable along each axis dimension.

As used herein, the term "linear" means a mathematical model based on the use of linear operators.

As used herein, the term "filter" means a linear operator is applied to a signal. This includes, but is not limited to: the operation of convolution (convolving) the signal with the 'filter kernel'; the operation of direct Fourier domain convolution by multiplication of the Fourier Transform of the signal by the complex conjugate of the Fourier Transform of the filter kernel; or other equivalent methods as described in standard texts (e.g. Oppenheim and Schafer, 'Discrete Time Signal Processing, Prentice Hall, ISBN 0-13-216771-9).

As used herein, the term "'kernel" means, a finite set of numbers that are convolved with the signal, for example by direct convolution (convolving); by Fourier domain convolution by multiplication of the Fourier Transform of the signal by the complex conjugate of the Fourier Transform of the filter kernel; or by other equivalent methods as described in standard texts (e.g. Oppenheim and Schafer, 'Discrete Time Signal Processing, Prentice Hall, ISBN 0-13-216771-9).

The term "filter kernel" as used herein may also referred to as the filter "coefficients" or the filter "impulse response" (Steven W Smith, "The Scientist's and Engineer's Guide to Digital Signal Processing", ISBN 0-9660176-4-1).

Preferably the design is such that the filters are separable along each axis dimension. Separability has the advantage to allow the design reconstruction filters in each dimension independently.

As used herein, the term "separable" means that the design of the nD reconstruction filter kernel may be done by separately designing 1D filter kernels along each of the original scan co-ordinate system's dimensions, and then combining these separate 1D filters by a process that is equivalent to: taking an nD set of kernel samples initialized to the value 1; and multiplying successively by each 1D filter kernel, in the direction of that kernel's dimension, to produce an nD kernel (Steven W Smith, "The Scientist's and Engineer's Guide to Digital Signal Processing", ISBN 0-9660176-4-1 for a definition of 'separability' as applied to filters). This process of combining separable 1D kernels to produce an nD kernel may be described by regarding the nD data set as an nD matrix and each 1D filter kernel as a 1D matrix (or vector), and successively calculating the Kronecker tensor product of each 1D matrix with the resulting nD matrix (Mary L Boas, "Mathematical Methods for the Physical Sciences", ISBN 0-471-04409-1).

The next step in the present method comprises: (f) applying to the sampled data said n-dimensional linear filter kernels (preferably applying said set of n-dimensional linear filter kernels), for example by direct convolution, for example using a polyphase implementation, which results in minimum computation, thereby obtaining re-sampled data.

As used herein, the term "convolution" means the integral over the interval of support of one function at x times another function at ( u - x ). In an embodiment, this may specifically mean the integral over the interval of support of the signal at x times the filter kernel at ( u - x ) (Steven W Smith, "The Scientist's and Engineer's Guide to Digital Signal Processing", ISBN 0-9660176-4-1).

In an embodiment, said method comprises re-sampling and interpolation of ultrasound data to a consistent n-dimensional sample space using n-dimensional (hereafter called nD) filters that are designed and applied in the natural space of the scan geometry, based on uniform sample spacing for both input and output samples relative to the co-ordinate axes of the original scan co-ordinate system when measured in units that are appropriate to that axis, by direct application of Sampling Theory so that the underlying signal remains completely determined.

In an embodiment, to re-sample the data while ensuring that the underlying signal remains completely determined, Sampling Theory requires that the data be filtered with anti-aliasing and reconstruction filters whose characteristics can be calculated readily from the original sample spacings and the target sample spacing, provided that both sets of spacings are uniform.

But in the target nD space the original samples are non-uniformly spaced, so Sampling Theory cannot be directly applied to design the required anti-aliasing and reconstruction filters in the target nD co-ordinate space.

However, the original samples are uniformly spaced in the original scan co-ordinate geometrical space, and so Sampling Theory may be directly applied in that original scan co-ordinate space to design filters that also must be applied in the original scan coordinate space. And a filter so designed and applied may be used to re-sample data by calculating new samples at desired uniform spacings in the new target co-ordinate space. This combined step (designing and implementing filters in the original scan co-ordinate space but applying them to calculate new samples that are uniformly spaced in the new target co-ordinate space) allows direct application of Sampling Theory which requires that samples be uniformly spaced.

According to said method, the positions of the desired target samples are non-uniform relative to the original scan co-ordinate system. So a position-quantization is applied, that approximates the position of all newly desired target samples to lie on a convenient and well defined position that is a simple and exact integer fraction of the spacing between original samples in the original scan co-ordinate system. For example newly desired sample positions may be quantized so that they are approximated by positions at ¼ or 1/5 of the original sample spacing. This position-quantization step allows direct application of Sampling Theory which requires that both input and output samples be uniformly spaced.

According to said method, an nD filter kernel can be designed by combining separable linear filters along each of the original scan dimensions, whose characteristics are determined according to the application of Sampling Theory based on the uniform sample rates of original and desired target samples.

According to said method, said nD filter kernel may be applied by direct convolution, by placing the filter kernel centered on each desired target re-sampled position and calculating the nD convolution product (sum of products). The position quantization step enforces that each nD filter coefficient does in fact coincide with an exact sample position in the position-quantized space. Further, the nD filter kernel can be implemented as an nD polyphase filter.

An embodiment of the present invention re-samples data from a 3D non-Cartesian scan geometry to a 3D rectangular Cartesian geometry.

An original sample is a point (zero dimensional - 0D), representing the signal at a single position in nD space.

For example an nD grid of original scan samples can be constructed from samples along beams that are projected by transducers using a beam-forming process.

A beam is a 1D line of samples. A beam is also commonly called a 'line of sight'.

An array of transducers projects a 2D frame of beams whose geometry is defined by the arrangement of transducers and the beam forming process.

When the frames are scanned by movement (rotation or translation) of the transducer array, or the transducer array is itself 2D, they form 3D scan that naturally defines three special dimensions, along each of which are samples that are uniformly spaced when measured in the units appropriate to that dimension (typically spatial distance, angle, or time).

A 4D scan may be done by measuring complete 3D volumes at intervals of time (time being the 4^{th} dimension in this example).

Each scan geometry can be represented by an n-dimensional co-ordinate system.

For example a 3D co-ordinate system, where each sample's position can be specified with a triplet of co-ordinates (A, B, C).

The 4D rectangular Cartesian co-ordinate system is represented by (x, y, z, t).

Some scanner geometries involve circular arrangements: either a rotational movement of the transducer array during scanning, or a circular shape to the transducer array: some have both.

Such geometries are best represented by polar co-ordinates. For example a linear array of transducers that is rotated about an axis parallel with its own line would define a cylindrical sample space that is best represented in cylindrical polar co-ordinates (r, theta, z) where z is transducer position, theta is the rotational angle, and r is the distance out along a beam.

A sample's position in a 1D, 2D, 3D or 4D space that is naturally defined by the scan geometry can be fixed by one, two, three or four co-ordinates:
Sample Position (along a beam): is the distance along the line of sight (LOS) of a beam; (original position of the sample along the line of sight of a beam (i.e. distance of the sample from the origin of the beam (sample position within a beam)),
Beam Position (along a sensor array) is the distance along the transducer line, or angle of the beam; compared to a reference central beam.

Frame Position (along a direction of scanning) is the distance along the scan line, or angle of the scan (a frame) compared to a reference central frame.

Time is the time of acquisition of a beam, frame or volume. Time may be the 2^{nd} 3^{rd} or 4^{th} dimension when applied to 1D, 2D or 3D spatial sample scans respectively.

Sample Position is usually a linear distance, while Beam Position (sometimes called 'line of sight' position) and Frame Position may be linear distances or angles depending on the scan geometry.
In a 2D spatial scan any individual sample's position is defined by the co-ordinates: (SamplePosition, BeamPosition)
In a 3D spatial scan any individual sample's position is defined by the triplet of coordinates: (SamplePosition, BeamPosition, FramePosition)
In a 4D space/time scan any individual sample's position is defined by the quadruplet of co-ordinates: (SamplePosition, BeamPosition, FramePosition, Time)

In one embodiment of the present invention for 2D spatial scans, said sample original coordinates are defined by two coordinates (Sample Position, Beam Position) as afore defined, and said nD linear filter kernels are 2D linear filter kernels.
In this embodiment, the position of a new re-sampled value can be given by its 2D rectangular Cartesian co-ordinates: (ResampleX, ResampleY)

In another embodiment of the present invention, said sample original coordinates are defined by three coordinates (Sample Position, Beam position, Frame Position) wherein Sample Position is the original position of the sample along the line of sight of a beam (distance of the sample from the origin of the beam (sample position within a beam), Beam position is the original distance or angle of a beam compared to a reference central beam, and Frame Position is the distance or the angle of a frame compared to a reference central frame, and said linear filter kernels are 3D linear filter kernels.

This embodiment comprises re-sampling of ultrasound scan data with filtering based on a 3D filter kernel volume, whose shape is defined naturally in the original scan geometry by an extension of each original scan coordinate and whose axes are naturally aligned with the original scan dimensions.

The position of a new re-sampled value can be given by its 3D rectangular Cartesian coordinates:
(ResampleX, ResampleY, ResampleZ)

In this re-sample geometry the new re-samples are spaced uniformly but the original scan samples are spaced non-uniformly.

The position of a new re-sampled value can also be given by its 3D co-ordinates in the original scan geometry:
(ResampleSamplePosition, ResampleBeamPosition, ResampleFramePosition)

The new re-sample does not necessarily coincide exactly with the position of any original scan sample, but the original samples are uniformly spaced along each of the original scan dimensions.

According to Sampling Theory the re-sample can be calculated by filtering and re-sampling along each of the original scan dimensions. This is equivalent to filtering with a 3D filter kernel, whose shape along each scan dimension is determined by the requirements of that dimension alone. (That is, the filters are separable in each original scan dimension).

In an embodiment, the 3D filter kernels are constructed by combining three separable re-sampling filters, each designed specifically for one original scan dimension, taking into account the special physical characteristics of the measurement along each scan dimension. Preferably, said 3D filter kernels are constructed by combining three separable re-sampling filters taking also into account of the requirements of Sampling Theory.

Because the filter kernel axes are aligned with the original scan dimensions, and those scan dimensions are orthogonal, the method successfully makes the 2D or 3D filter design separable into two or three 1D filter designs, one for each dimension, which greatly simplifies the filter design.

The method correctly applies specific filtering in the special directions imposed by the original scan geometry, to maximally retain complete determination of the underlying continuous ultrasound signal that was represented by the original samples, according to the requirements of Sampling Theory.

The re-sampling in each dimension requires a filter whose coefficients are designed with a filter sample rate that is determined by the distance between the new re-sample and the nearest original sample along that dimension. Many filter sample rates may result: in an embodiment, the method of the present invention limits this number by quantizing the possible positions of the re-sample, as specified in the original scan dimensions, to a (small) multiple of the original scan sample rate along that dimension. This multiple is called the FilterRateMultiplier and may be a variable parameter.

In yet another embodiment, the sample original coordinates are defined by four coordinates (Sample Position, Beam position, Frame Position, time) wherein Sample Position is the original position of the sample along the line of sight of a beam (distance of the sample from the origin of the beam (sample position within a beam), Beam position is the original distance or angle of a beam compared to a reference central beam, and Frame Position is the distance or the angle of a frame compared to a reference central frame, and said linear filters are 4D linear filter kernels. In an embodiment, the method for re-sampling applies 3D filter kernels based on the values from neighboring original scan samples. The filter shape is designed based on considering: Sampling Theory (anti-aliasing) along each of the three scan dimensions; physics (ultrasound wavelength represents a fundamental limit to practical resolution); and normalization (a DC signal's value should be the same after filtering).

In an embodiment, said linear filters are anti-aliasing filters.

In a preferred embodiment, said linear filters are low pass digital FIR filters.

In an embodiment, the low pass-filters are specified through the following parameters: stopband, a passband, and a stopband attenuation. Preferably the low pass-filters are specified through the following parameters: a sample rate, a stopband, a passband, a passband ripple and a stopband attenuation. In an embodiment, the passband must be lower than that required by Sampling Theory based on the input and output sample rates in each dimension. The stopband attenuation is the degree to which disallowed frequencies are in fact rejected (as required by Sampling Theory for the underlying signal to be completely determined).

In a preferred embodiment the low-pass filters for use in the present method are specified through five parameters:
■ sample rate: the sample rate for implementation
■ passband: the frequency up to which values are 'unchanged'
■ passband ripple: the maximum amount of change we allow within the passband
■ stopband: the frequency above which components are attenuated
■ stopband attenuation: the minimum attenuation above the stopband

The present invention has the advantages of simplifying the display, extraction of features from, and analysis of 3D ultrasound scan data by providing a 3D Cartesian sample space that is convenient to process and that reduces the amount of data to be processed without undue loss of genuine information.

The present invention also provides an apparatus for re-sampling ultrasound scan data comprising a 3D filter kernel design module, a 3D re-sampling and filter kernel implementation module and a 3D re-sampling module. Said apparatus can be one or more processors configured to design a 3D filter kernel, to implement 3D re-sampling and filtering. Said processors can be provided in one computer or in two or more computers.

The present invention also provides an ultrasound processing system, comprising:
(a) at least one means for acquiring reflected or transmitted ultrasound scan data; (b) at least one sampling module, and (c) at least one processor comprising a 3D re-sampling module, said processor being configured to design a 3D filter kernel, and implement 3D resampling and filtering.

In an embodiment, said sampling module is comprised in said at least one processor.

The present method, system and apparatus has the advantages of simplifying the analysis, design and implementation of 3D re-sampling filters for 3D ultrasound scans by doing the design and implementation in the original scan geometry where the scan dimensions and therefore coordinate axes are orthogonal and the samples are uniformly spaced (so that Sampling Theory may be readily applied).

The present invention permits rigorous design of re-sampling filters according to Sampling Theory, because the scan dimensions and therefore coordinate axes are orthogonal and the samples are uniformly spaced.

The present method and apparatus reduces the amount of data to be processed without undue loss of genuine information, because it respects the requirements of Sampling Theory for each original scan dimension specifically and correctly applies a priori knowledge from the physical characteristics of the ultrasound scanning process to choose the minimum of data that adequately represents the original genuine information that is encapsulated in the scan data. In an embodiment, the amount of data is reduced by a factor of 5.

The present invention allows the sampled data to be further processed and analyzed without distortion or loss of information, because the underlying signal remains completely determined. This is a benefit in any case where measurements such as characterization of tissue rely for their efficacy on processing or analysis that relies on an accurate determination of the underlying signal. In contrast, in prior art the underlying signal may not remain completely determined by the samples because the filters cannot easily be designed by direct application of Sampling Theory. Also, prior art methods design re-sampling filters in the desired target co-ordinate system, in which the original samples are spaced non-uniformly and so filter design using Sampling Theory (which assumes uniform sample spacing) is not possible: so these filters cannot be shown not to result in distortion or loss of information.

For example the present method can retain maximum information for minimum data size, and so allows storage requirements and computation to be kept to a minimum. This is because the minimum number of samples necessary to completely determine the underlying signal, and the necessary filter designs, can be calculated directly by application of Sampling Theory and filter length estimation (for example Kaiser's method). In contrast with the present invention, prior art cannot directly apply Sampling Theory to calculate the minimum number of samples necessary to completely determine the underlying signal, so that either more samples may be used than are necessary, or the signal may be distorted or information may be lost. In this way the present invention permits a formal analysis of the minimum data size required, which can both reduce storage requirements and reduce scan time.

For example the present invention facilitates re-sampling ultrasound scan data from various original n-dimensional scan geometries and sample spaces, to a uniform n-dimensional sample grid (often, but not only, rectangular Cartesian) that is convenient for further processing and that reduces the amount of data while retaining the original information according to the measures of Sampling Theory and the physical characteristics of the ultrasound scanning process. The present invention greatly simplifies the display, extraction of features from, and analysis of n-dimensional ultrasound scan data by providing an n-dimensional sample space that is convenient to process and that reduces the amount of data to be processed without undue loss of genuine information.

The invention will now be illustrated by means of the following example, which does not limit the scope of the invention in any way.

### Example

The filtering and re-sampling according to an embodiment of the present invention is based on the same filter being applied in all directions. This has the benefit that the re-sampling is independent of the scan geometry, and it takes account of all sample rates present in the original data. It defines a spherical filter kernel (the kernel is the array of filter coefficients, in this case a 3D array).

The present example is based on prostate ultrasound scanning. The present example casts the re-sampled positions into the original geometry then design and applies the filters in that original geometry.

The position of each original sample in the prostate scan can be given by three coordinates in a spherical polar space: (SamplePos, BeamPos, FramePos)
wherein:
SamplePos is line of sight distance (in mm) from the (imagined) origin of the beams BeamPos is the angle (in radians) of the beam relative to the 'central' beam
FramePos is the angle (in radians) of the frame relative to the 'central' frame
The position of the new re-sampled value can be given by its 3D rectangular Cartesian co-ordinates: (x, y, z) but can also be defined in spherical polar co-ordinates: (r, theta, phi)
In this spherical polar geometry the original samples are uniformly spaced along each of the dimensions. This re-sample lies on a virtual Line Of Sight that radiates out from the beam origin.

The angle of this beam is defined by (theta, phi) and neither theta nor phi need necessarily match any original BeamPos or FramePos respectively. The radial distance of the re-sample is defined by r and r does not necessarily match any original SamplePos.

A 3D filter kernel is designed whose dimensions are set by three filters: filter along the line of sight, filter along the beam spacing and filter along the frame spacing.

The three dimensions of required filtering and re-sampling naturally define a 'quadrilateral pyramid' geometry: (r, theta, phi) and the 3D filter kernel is a slice through this quadrilateral pyramid.

The height of the pyramid slice is the length of the Line Of Sight filter and is fixed. The two other dimensions are fixed by the lengths of the inter-frame and inter-beam filters: in these two dimensions the spacings are angles.

The re-sampling process can be seen as applying a quadrilateral pyramid filter that applies weights to the surrounding original samples to calculate the new re-sample that is at the centre of the pyramid. But it can also be thought of as re-sampling along each of the original scan dimensions: in which case we have three separate 1-D filters, one for each re-sampling. These three 1-D filters can then be combined to create the necessary 3D quadrilateral pyramid filter.

When calculating the new samples two cases could be seen: up-sampling (where more samples than were originally measured should be interpolated) and down-sampling (where some of the original samples should be discarded). In both cases the signal should be filtered with a low-pass filter.

The low-pass filter used in the present invention eliminates frequency components that cannot be present. To down-sample the original samples are filtered with a low-pass filter, then some of the original samples are discarded. The low-pass filter for down-sampling eliminates frequencies that are higher than half the new sample rate. This means that the cut-off may vary anywhere up to half the original sample rate. To up-sample zeroes are interpolated at the new (higher sample rate) sample locations, then filtered with a low-pass filter. The low-pass filter for up-sampling eliminates frequencies that are higher than half the original sample rate. This cut-off is fixed regardless of the new up-sampled rate.

For the prostate scans, three directions for the filters apply: along the Line Of Sight (which is a distance in mm) and two directions across the Line Of Sight (which are angles in radians in this example).

The low-pass digital FIR filter for use in the present examples can be specified through five parameters:

| | |
|---|---|
| sample rate | the sample rate for implementation |
| passband: | the frequency up to which values are 'unchanged' |
| passband ripple: | the maximum amount of change we allow within the passband |
| stopband: | the frequency above which components are attenuated |

| | |
|---|---|
| stopband attenuation: the minimum attenuation above the stopband | |

The lowest original, and the new re-sampled, sample rates set the bounds on the present filter design as follows:

| | |
|---|---|
| lowest original sample rate: | lowest required cut-off |
| re-sample rate: | highest required cut-off |

### Line Of Sight filter

The Line Of Sight filter is determined by the Line Of Sight sample spacing and the re-sample spacing. For prostate scans the Line Of Sight sample spacing is fixed, at 0.044 mm which is a sample rate of 22.7 samples per mm. the re-sample spacing is 0.200 mm which is a sample rate of 5 cycles/mm, so we are down-sampling by 4.54.

In this case the sample spacing and the re-sample spacing are both fixed and the re-sample rate is the lower, so it fixes the required cut-off:

| | |
|---|---|
| Line Of Sight cut-off | = 2.50 cycles/mm |
| Line Of Sight filter sample rate | = 22.7 samples/mm |

The passband width is of 1 sample/mm, making a passband of 2 cycles/mm. Adopting this cut-off with the previously recommended parameters

| | |
|---|---|
| passband: | 2 |
| stopband: | 2.5 |
| passband ripple: | 6 dB |
| stopband attenuation = | 20 dB |
| sample rate = | 22.7 samples/mm |

and designing an equiripple filter using fdatool, provides a filter that needs 8 coefficients.

### Inter-beam and inter-frame filter

The inter-beam filter is determined by the beam spacing and the re-sample angle spacing. The narrowest low-pass filter is required when up-sampling and its passband is determined by the inter-beam spacing.

The parameters are:
re-sample angle sample rate = 500 samples/radian
beam rate = 200 beams/radian
frame rate = 333 frames/radian

These spacing mean that up-sampling will happen in these radial directions (by factors of 2.5 and 1.5).

A passband that is 10% of the stopband is set.

For the inter-beam filter (that is, for beams within a single frame) the filter can be specified as:

| | |
|---|---|
| passband: | 90 samples/radian |
| stopband: | 100 samples/radian |
| passband ripple: | 6 dB |
| stopband attenuation = | 20 dB |
| sample rate = | 500 samples/radian |

and for the inter-frame filter (that is, between adjacent frames):

| | |
|---|---|
| passband: | 150 samples/radian |
| stopband: | 166 samples/radian |
| passband ripple: | 6 dB |
| stopband attenuation = | 20 dB |
| sample rate = | 500 samples/radian |

These provide filters that need 8 and 5 coefficients respectively.

### Filter kernel

The filter kernel is 3D. It can be calculated by modulating in each dimension by the filter coefficients. For example:
1. by filling all the columns in the line of sight dimension with the line of sight coefficients
2. multiplying across line of sight in beam direction by the beam filter, and
3. multiplying across line of sight in the frame dimension by the frame filter.

This creates a 3D matrix of filter coefficients. These can be applied as weights to the surrounding samples when calculating a new re-sample value.

While the details of the present invention have been described with specific reference to a preferred embodiment, it is apparent that variations and applications may be made without departing from the spirit and scope of the inventive concept as defined by the appended claims.

## Claims

1. Method of re-sampling ultrasound scan data, comprising the steps of:
a) obtaining sampled ultrasound scan data acquired from a beamforming system, the sampled data being defined by an original n-dimensional sample coordinate system having n axes, that is defined by the ultrasound probe and scan geometry and in which the samples are spaced uniformly along each axis when measured in units appropriate to that axis;
b) defining desired target sample positions in a target n-dimensional co-ordinate system, that are uniformly spaced along each axis when measured in units appropriate to that axis;
c) mapping the target sample positions defined in step (b) into said original n-dimensional sample co-ordinate system of step (a);
**characterized by** comprising the further steps of:
d) quantizing the positions of the mapped target samples of step (c) so that they fall on simple exact integer subspacings between the original sample positions;
e) designing n-dimensional linear filter kernels according to application of Nyquist-Shannon Sampling Theory, and using the original sample coordinates of the sampled data of step (a) and the desired target sample positions of step (d) in their respective n-dimensional spaces, optionally said n-dimensional filters being separable along each of the original scan dimensions; and
f) applying to the sampled data of step (a) the n-dimensional linear filter kernels designed in step (e), each filter being applied to calculate the target sample thereby obtaining re-sampled data.

2. The method according to claim 1, wherein said n-dimensional filters are separable along each of the original scan dimensions.

3. The method according to claim 1 or 2, wherein said step (f) is performed by direct convolution.

4. The method according to any of claims 1 to 3, wherein said step (f) is performed using a polyphase implementation.

5. The method according to any of claims 1 to 3, wherein the sample original coordinates are defined by two coordinates, the coordinates being Sample Position and Beam position, wherein Sample Position is the original position of the sample along the line of sight of a beam , Beam position is the original distance or angle of a beam compared to a reference central beam, and said n-dimensional linear filter kernels are 2D linear filter kernels.

6. The method according to any of claims 1 to 3, wherein said sample original coordinates are defined by three coordinates, the coordinates being Sample Position, Beam position and Frame Position, wherein Sample Position is the original position of the sample along the line of sight of a beam , Beam position is the original distance or angle of a beam compared to a reference central beam, and Frame Position is the distance or the angle of a frame compared to a reference central frame, and said n-dimensional linear filter kernels are 3D linear filter kernels.

7. The method according to claim 6, wherein the 3D filter kernels are constructed by combining three separable re-sampling filters, each designed specifically for one original scan dimension, taking account of the physical characteristics of the measurement along each scan dimension.

8. The method according to any of claims 1 to 3, wherein the sample original coordinates are defined by four coordinates, the coordinates being Sample Position, Beam position, Frame Position and time, wherein Sample Position is the original position of the sample along the line of sight of a beam , Beam position is the original distance or angle of a beam compared to a reference central beam, and Frame Position is the distance or the angle of a frame compared to a reference central frame, and said linear filter kernels are 4D linear filter kernels.

9. The method according to any one of claims 1 to 8, wherein said linear filters are anti-aliasing filters.

10. The method according to any one of claim 1 to 9, wherein said linear filters are low pass digital FIR filters.

11. The method according to claim 10, wherein said low pass-filters are specified through the following parameters:a stopband, a passband and a stopband attenuation.

12. The method according to claim 11, wherein said low-pass filters are further specified through the following parameters: the sample rate and a passband ripple.

13. The method according to any one of claim 1 to 12, wherein a set of n-dimensional linear filter kernels is designed, one for each different target sample position relative to the original sample positions of its nearest neighbors.

14. An apparatus for re-sampling ultrasound scan data comprising a 3D filter kernel design module, a 3D re-sampling and filter kernel implementation module and a 3D re-sampling module, whereby the apparatus is configured to perform the method of re-sampling ultrasound scan data according to any of the claims 1 to 13.

15. An ultrasound processing system, comprising :
(a) at least one means for acquiring scattered, reflected or transmitted ultrasound scan data;
(b) at least one sampling module,
(c) at least one processor comprising a 3D re-sampling module, said processor being configured to
design a 3D filter kernel, and
implement 3D re-sampling and filtering,
whereby said processor is configured to perform the method of re-sampling ultrasound scan data according to any of the claims 1 to 13.

## Patentansprüche

1. Ein Verfahren für die Wiederabtastung von Ultraschallsignaldaten, folgende Schritte umfassend:
a) das Einholen von abgetasteten Ultraschallsignaldaten, die durch ein Strahlformungssystem erworben wurden, wobei die abgetasteten Signaldaten von einem ursprünglichen n-dimensionalen Abtast-Koordinatensystem mit n Achsen definiert sind, welches seinerseits von der Ultraschallsonde und der Signalgeometrie definiert ist und in dem die abgetasteten Signaldaten gleichmäßig entlang jeder Achse abstandsgleich folgen, wenn in geeigneten Einheiten zu dieser Achse gemessen;
b) die Definition der gewünschten gezielten Abtast-Positionen in einem gezielten n-dimensionalen Koordinatensystem, in welchem sie gleichmäßig entlang jeder Achse abstandsgleich folgen, wenn in geeigneten Einheiten zu dieser Achse gemessen;
c) die Abbildung der im Schritt (b) definierten gezielten Abtast-Positionen in dem ursprünglichem, im Schritt (a) beschriebenen n-dimensionalen Abtast-Koordinatensystem, durch die Umfassung der folgenden weiteren Schritte gekennzeichnet;
d) das Quantisieren der im Schritt (c) abgebildeten, gezielten Abtast-Positionen, so dass sie auf einfache genaue Ganzzahl-Unterabstände zwischen den ursprünglichen abgetasteten Positionen fallen;
e) der Entwurf n-dimensionaler linearen Filterkerne nach Anwendung des Nyquist-Shannons Abtasttheorem, unter Nutzung der ursprünglichen abgetasteten Koordinaten der im Schritt (a) abgetasteten Daten und der im Schritt (d) gewünschten gezielten Abtast-Positionen in deren jeweiligen n-dimensionalen Räume, wobei die besagten n-dimensionale Filter wahlweise entlang jeder der ursprünglichen Signaldimensionen trennbar sind; und
f) die Anwendung der im Schritt (e) entworfen n-dimensionalen linearen Filterkerne auf die im Schritt (a) abgetasteten Daten, wobei jeder Filter angewendet wird, um die Zielabtastung zu berechnen, dadurch Wiederabtastungsdaten bekommend.

2. Das Verfahren nach Anspruch 1, wobei die besagten n-dimensionalen Filter entlang jeder der ursprünglichen Signaldimensionen, trennbar sind.

3. Das Verfahren nach einem der Ansprüche 1 oder 2, wobei der besagte Schritt (f) durch eine direkte Faltung durchgeführt wird.

4. Das Verfahren nach einem der Ansprüche 1 bis 3 , wobei der Schritt (f) durch eine mehrphasige Anwendung durchgeführt wird.

5. Das Verfahren nach einem der Ansprüche 1 bis 3, wobei die ursprünglichen abgetasteten Koordinaten durch zwei Koordinaten definiert sind, wobei diese Koordinaten die Abtast und die Strahlposition sind, wobei die abgetastete Signalposition die ursprüngliche Position der abgetasteten Position entlang der Sichtlinie eines Strahls ist, und die Strahlposition der ursprüngliche Abstand oder Winkel eines Strahls im Vergleich zu einem zentralen Referenzstrahl ist, und die besagten n-dimensionale lineare Filterkerne, 2D-lineare Filterkerne sind.

6. Das Verfahren nach einem der Ansprüche 1 bis 3, wobei die besagten ursprünglichen abgetasteten Koordinaten durch drei Koordinaten definiert sind, wobei die Koordinaten die abgetastete Signalposition, die Strahlposition und die Rahmenposition sind, wobei die abgetastete Signalposition die ursprüngliche Position der Abtast entlang der Sichtlinie eines Strahls ist, die Strahlposition der ursprüngliche Abstand oder Winkel eines Strahls im Vergleich zu einem zentralen Referenzstrahl ist, und die Rahmenposition der Abstand oder der Winkel eines Rahmens im Vergleich zu einem zentralen Referenzrahmen ist, und die besagten n-dimensionale lineare Filterkerne, 3D-lineare Filterkerne sind.

7. Das Verfahren nach Anspruch 6, wobei die 3D-Filterkerne durch die Kombination von drei trennbaren Wiederabtastungsfilter gebildet sind, jeweils spezifisch für eine ursprüngliche Abtastungsdimension ausgelegt, unter Berücksichtigung der physikalischen Eigenschaften der Messung entlang jeder Abtastungsdimension.

8. Das Verfahren nach einem der Ansprüche 1 bis 3, wobei die ursprünglichen abgetasteten Koordinaten durch vier Koordinaten definiert sind, wobei diese Koordinate die abgetastete Signalposition, die Strahlposition, die Rahmenposition und die Zeit sind wobei die abgetastete Signalposition die ursprüngliche Position der Abtast entlang der Sichtlinie eines Strahls ist, die Strahlposition der ursprüngliche Abstand oder Winkel eines Strahls im Vergleich zu einem Referenzzentralen Strahl ist, und die Rahmenposition der Abstand oder der Winkel eines Rahmens im Vergleich zu einem zentralen Referenzrahmen ist, und die besagten lineare Filterkerne, 4D-lineare Filterkerne sind.

9. Das Verfahren nach einem der Ansprüche 1 bis 8, wobei die besagten lineare Filter, Anti-Aliasing-Filter sind.

10. Das Verfahren nach einem der Ansprüche 1 bis 9, wobei die besagten lineare Filter, digitale Tiefpass-FIR-Filter sind.

11. Das Verfahren nach Anspruch 10, wobei die besagten Tiefpass-Filter durch die folgenden Parameter bestimmt werden: ein Sperrbereich, ein Durchlassbereich und eine Sperrdämpfung.

12. Das Verfahren nach Anspruch 11, wobei die besagten Tiefpassfilter weiter durch die folgenden Parameter bestimmt werden: die Abtastrate und eine Welligkeit im Durchlassbereich.

13. Das Verfahren nach einem der Ansprüche 1 bis 12, wobei ein Satz von n-dimensionalen linearen Filterkernen, jeweils ein Filterkern für jede gezielte Abtastposition relativ zu den ursprünglichen abgetasteten Positionen seiner nächsten Nachbarn, ausgelegt ist.

14. Eine Vorrichtung zur Wiederabtastung von Ultraschallsignaldaten, umfassend einem 3D-Filterkern Gestaltungsmodul, einem 3D-Wiederabtastungs- und Filterkern-Implementierungsmodul und ein 3D-Wiederabtastungsmodul, wobei die Vorrichtung konfiguriert ist, um das Verfahren der Wiederabtastung von Ultraschallsignaldaten gemäß einem der Ansprüche 1 bis 13 auszuführen.

15. Ein Ultraschall-Verarbeitungssystem, umfassend:
(a) mindestens ein Mittel zum Erfassen von gestreuten, reflektierten oder übertragenen Ultraschallsignaldaten;
(b) mindestens ein Abtastmodul,
(c) mindestens einen Prozessor umfassend einem 3D-Wiederabtastungsmodul, wobei der Prozessor konfiguriert ist, um einen 3D-Filterkern zu entwerfen und die 3D-Wiederabtastung und das Filtern auszuführen, wobei der besagte Prozessor konfiguriert ist, um das Verfahren der Wiederabtastung von Ultraschallsignaldaten gemäß einem der Ansprüche 1 bis 13 auszuführen.

## Revendications

1. Procédé de rééchantillonnage de données d'échographie, comprenant les étapes suivantes :
a) obtention de données d'échographie échantillonnées acquises à partir d'un système de formation de faisceau, les données échantillonnées étant définies par un système de coordonnées d'échantillons à n dimensions d'origine ayant n axes, qui est défini par la sonde à ultrasons et la géométrie de balayage, et dans lequel les échantillons sont espacés uniformément le long de chaque axe lorsqu'ils sont mesurés dans des unités appropriées à cet axe ;
b) définition de positions d'échantillons cibles souhaitées dans un système de coordonnées à n dimensions cible, qui sont espacées uniformément le long de chaque axe lorsqu'elles sont mesurées dans des unités appropriées à cet axe ;
c) mappage des positions d'échantillons cibles définies à l'étape (b) dans ledit système de coordonnées d'échantillons à n dimensions d'origine de l'étape (a) ; **caractérisé par** le fait de comprendre les étapes supplémentaires suivantes :
d) quantification des positions des échantillons cibles mappées de l'étape (c) de sorte qu'elles tombent sur des sous-espacements entiers exacts simples entre les positions d'échantillons d'origine ;
e) conception de noyaux de filtres linéaires à n dimensions en fonction de l'application du Théorème d'Echantillonnage de Nyquist-Shannon, et utilisation des coordonnées d'échantillons d'origine des données échantillonnées de l'étape (a) et des positions d'échantillons cibles souhaitées de l'étape (d) dans leurs espaces à n dimensions respectifs, éventuellement lesdits filtres à n dimensions étant séparables le long de chacune des dimensions de balayage d'origine ; et
f) application aux données échantillonnées de l'étape (a) des noyaux de filtres linéaires à n dimensions conçus à l'étape (e), chaque filtre étant appliqué pour calculer l'échantillon cible de façon à obtenir des données rééchantillonnées.

2. Procédé selon la revendication 1, dans lequel lesdits filtres à n dimensions sont séparables le long de chacune des dimensions de balayage d'origine.

3. Procédé selon la revendication 1 ou 2, dans lequel ladite étape (f) est exécutée par convolution directe.

4. Procédé selon l'une quelconque des revendications 1 à 3, dans lequel ladite étape (f) est exécutée en utilisant une mise en oeuvre polyphasée.

5. Procédé selon l'une quelconque des revendications 1 à 3, dans lequel les coordonnées d'échantillons d'origine sont définies par deux coordonnées, les coordonnées étant la Position de l'Echantillon et la Position du Faisceau, dans lequel Position de l'Echantillon est la position d'origine de l'échantillon le long de la ligne de visée d'un faisceau, la Position du Faisceau est la distance ou l'angle d'origine d'un faisceau par rapport à un faisceau central de référence, et lesdits noyaux de filtres linéaires à n dimensions sont des noyaux de filtres linéaires 2D.

6. Procédé selon l'une quelconque des revendications 1 à 3, dans lequel lesdites coordonnées d'échantillons d'origine sont définies par trois coordonnées, les coordonnées étant la Position de l'Echantillon , la Position du Faisceau et la Position du Cadre, dans lequel la Position de l'Echantillon est la position d'origine de l'échantillon le long de la ligne de visée d'un faisceau, la Position du Faisceau est la distance ou l'angle d'origine d'un faisceau par rapport à un faisceau central de référence, et la Position du Cadre est la distance ou l'angle d'un cadre par rapport à un cadre central de référence, et lesdits noyaux de filtres linéaires à n dimensions sont des noyaux de filtres linéaires 3D.

7. Procédé selon la revendication 6, dans lequel les noyaux de filtres 3D sont construits en combinant trois filtres de rééchantillonnage séparables, chacun étant conçu spécifiquement pour une dimension de balayage d'origine, en tenant compte des caractéristiques physiques de la mesure le long de chaque dimension de balayage.

8. Procédé selon l'une quelconque des revendications 1 à 3, dans lequel les coordonnées d'échantillons d'origine sont définies par quatre coordonnées, les coordonnées étant la Position de l'Echantillon , la Position du Faisceau, la Position du Cadre, et le temps, dans lequel la Position de l'Echantillon est la position d'origine de l'échantillon le long de la ligne de visée d'un faisceau, la Position du Faisceau est la distance ou l'angle d'origine d'un faisceau par rapport à un faisceau central de référence, et la Position du Cadre est la distance ou l'angle d'un cadre par rapport à un cadre central de référence, et lesdits noyaux de filtres linéaires sont des noyaux de filtres linéaires 4D.

9. Procédé selon l'une quelconque des revendications 1 à 8, dans lequel lesdits filtres linéaires sont des filtres anti-repliement.

10. Procédé selon l'une quelconque des revendications 1 à 9, dans lequel lesdits filtres linéaires sont des filtres FIR numériques passe-bas.

11. Procédé selon la revendication 10, dans lequel lesdits filtres passe-bas sont spécifiés par les paramètres suivants : une bande atténuée, une bande passante et une atténuation de la bande atténuée.

12. Procédé selon la revendication 11, dans lequel lesdits filtres passe-bas sont en outre spécifiés par les paramètres suivants : la fréquence d'échantillonnage et une ondulation dans la bande passante.

13. Procédé selon l'une quelconque des revendications 1 à 12, dans lequel un ensemble de noyaux de filtres linéaires à n dimensions est conçu, un pour chaque position d'échantillon cible différente par rapport aux positions d'échantillons d'origine de ses voisins les plus proches.

14. Appareil pour le rééchantillonnage de données d'échographie comprenant un module de conception de noyaux de filtres 3D, un module de mise en oeuvre de rééchantillonnage et de noyaux de filtres 3D et un module de rééchantillonnage 3D, moyennant quoi l'appareil est configuré pour exécuter le procédé de rééchantillonnage de données d'échographie selon l'une quelconque des revendications 1 à 13.

15. Système de traitement d'ultrasons, comprenant:
(a) au moins un moyen pour acquérir des données d'échographie dispersées, réfléchies ou transmises ;
(b) au moins un module d'échantillonnage,
(c) au moins un processeur comportant un module de rééchantillonnage 3D, le processeur étant configuré pour concevoir un noyau de filtre 3D, et mettre en oeuvre un rééchantillonnage et un filtrage 3D, moyennant quoi ledit processeur est configuré pour exécuter le procédé de rééchantillonnage de données d'échographie selon l'une quelconque des revendications 1 à 13.
